# EUROPEAN PATENT SPECIFICATION

(11) **EP 1 868 599 B1**
(45) Date of publication and mention of the grant of the patent: **11.11.2009**
(21) Application number: 06725409.4
(22) Date of filing: 29.03.2006
(51) Int. Cl.: A61K 31/40, A61K 39/395, A61P 35/00

(54) **ANTITUMOR COMBINATION COMPRISING SUBSTITUTED ACRYLOYL DISTAMYCIN DERIVATIVES AND ANTIBODIES INHIBITING GROWTH FACTORS OR THEIR RECEPTORS**
ANTITUMORALE KOMBINATION AUS SUBSTITUIERTEN ACRYLOYLDISTAMYCIN-DERIVATEN UND WACHSTUMSFAKTOREN ODER IHRE REZEPTOREN HEMMENDE ANTIKÖRPER
COMBINAISON ANTITUMORALE COMPRENANT DES DERIVES ACRYLOYL DISTAMYCINE SUBSTITUES ET ANTICORPS INHIBANT LES FACTEURS DE CROISSANCE DE LEURS RECEPTEURS

(30) Priority: 08.04.2005 EP 05102792
(43) Date of publication of application: 26.12.2007
(73) Proprietor: NERVIANO MEDICAL SCIENCES S.r.l., 20014 Nerviano (MI) (IT)
(72) Inventor: PESENTI, Enrico, I-20015 Parabiago (IT); CIOMEI, Marina, I-20094 Corsico (IT); GERONI, Maria Cristina, I-20149 Milan (IT)
(74) Representative: Mazzini, Giuseppe
(86) International application number: PCT/EP2006/061153
(87) International publication number: WO 2006/108763

(56) References cited:
- WO-A-03/082267
- SPICER J: "Technology evaluation: Nimotuzumab, The Center of Molecular Immunology/YM BioSciences/Oncoscience" CURRENT OPINION IN MOLECULAR THERAPEUTICS 2005 UNITED KINGDOM, vol. 7, no. 2, 2005, pages 182-191, XP009069288 ISSN: 1464-8431
- GRAEVEN U ET AL: "Phase I study of the humanised anti-EGFR monoclonal antibody matuzumab (EMD 72000) combined with gemcitabine in advanced pancreatic cancer." BRITISH JOURNAL OF CANCER. 8 MAY 2006, vol. 94, no. 9, 8 May 2006 (2006-05-08), pages 1293-1299, XP002389797 ISSN: 0007-0920
- XIONG H Q ET AL: "CETUXIMAB, A MONOCLONAL ANTIBODY TARGETING THE EPIDERMAL GROWTH FACTOR RECEPTOR, IN COMBINATION WITH GEMCITABINE FOR ADVANCED PANCREATIC CANCER: A MULTICENTER PHASE II TRIAL" JOURNAL OF CLINICAL ONCOLOGY, GRUNE AND STRATTON, NEW YORK, NY, US, vol. 22, no. 13, 1 July 2004 (2004-07-01), pages 2610-2616, XP009061496 ISSN: 0732-183X
- BASELGA J: "CURRENT AND PLANNED CLINICAL TRIALS WITH TRASTUZUMAB (HERCEPTIN)" SEMINARS IN ONCOLOGY, W.B. SAUNDERS,, US, vol. 29, no. 5, SUPPL 9, October 2000 (2000-10), pages 27-32, XP002201756 ISSN: 0093-7754
- FREEMAN D ET AL: "313 Mono- and combination-therapeutic activity of panitumumab (ABX-EGF) on human A431 epidermoid and HT-29 colon carcinoma xenografts: correlation with pharmacodynamic parameters" EUROPEAN JOURNAL OF CANCER. SUPPLEMENT, PERGAMON, OXFORD, GB, vol. 2, no. 8, September 2004 (2004-09), pages 95-96, XP004639757 ISSN: 1359-6349
- COBLEIGH M A ET AL: "A PHASE I/II DOSE-ESCALATION TRIAL OF BEVACIZUMAB IN PREVIOUSLY TREATED METASTATIC BREAST CANCER" SEMINARS IN ONCOLOGY, W.B. SAUNDERS,, US, vol. 30, no. 5, SUPPL 16, October 2003 (2003-10), pages 117-124, XP009067366 ISSN: 0093-7754
- VALLE J W ET AL: "287 A phase Ib study of pertuzumab (P), a recombinant humanized antibody to HER2, and capecitabine (C) in patients with advanced solid tumors" EUROPEAN JOURNAL OF CANCER. SUPPLEMENT, PERGAMON, OXFORD, GB, vol. 2, no. 8, September 2004 (2004-09), page 88, XP004639731 ISSN: 1359-6349

## Description

The present invention relates to the field of cancer treatment and provides an antitumor combination comprising a substituted acryloyl distamycin derivative, more particularly α-bromo- or α-chloro-acryloyl distamycin derivative, and an antibody inhibiting a growth factor or its receptor, having a synergistic antineoplastic effect.

Distamycin A and analogues thereof, hereinafter referred to as distamycin and distamycin-like derivatives, are known in the art as cytotoxic agents useful in antitumor therapy.

Distamycin A is an antibiotic substance with antiviral and antiprotozoal activity, having a polypyrrole framework [Nature 203: 1064 (1964); J. Med. Chem. 32: 774-778 (1989)]. The international patent applications WO 90/11277, WO96/05196, WO 98/04524, WO 98/21202, WO 99/50265, WO 99/50266 and WO 01/40181 disclose acryloyl distamycin derivatives wherein the amidino moiety of distamycin is optionally replaced by nitrogen-containing ending groups such as, for instance, cyanamidino, N-methylamidino, guanidino, carbamoyl, amidoxime, cyano and the like, and/or wherein the polypyrrole framework of distamycin, or part of it, is replaced by varying carbocyclic or heterocyclic moieties.

Monoclonal antibodies (mABs) against growth factors or their receptors have been revealed to be effective therapeutic agents in antitumor therapy [see for a reference, Cancer Sci. 95: 621-25, (2004); Curr.Mol.Med 4: 539-47, (2004)].

Multiple mechanisms of monoclonal antibody action are being exploited for this purpose. Antibodies can sequester growth factors and prevent the activation of crucial growth factor receptors. A monoclonal antibody directed against the vascular endothelial growth factor (VEGF) has been shown to be a potent neo-vascularisation inhibitor (bevacizumab). An antibody against the extracellular domain of the epidermal growth factor (EGF) receptor prevents the binding of the ligand to the receptor and thereby its activation (cetuximab). EGFR activity, however, is absolutely required for the survival and proliferation of certain human tumour cells. An antibody which interferes with the dimerisation of the ErbB2 and the ErbB3 members of the EGF receptor family prevents the association of a most potent signaling module (pertuxumab). The signals emenating from this dimer determine many phenotypic properties of e.g. human breast cancer cells. A monoclonal antibody also directed against ErbB2 (an oncogene that encodes a receptor tyrosine kinase of the EGF-receptor family) has been most successful, clinically and commercially (Trastuzumab). This antibody interferes with signals generated by the receptor and causes the arrest of the cell cycle in tumour cells. A selection of these agents is shown in Table 1.

**Table 1: Antibodies inhibiting growth factors or their receptors in clinical development**

| **Target** | **Name** |
|---|---|
| VEGF | Bevacizumab |
| EGF-R | Cetuximab |
| | Panitumumab |
| | Matuzumab |
| | Nimotuzumab |
| ErbB-2 | Trastuzumab |
| | Pertuzumab |

It has now been surprisingly found that the antitumor effect of an acryloyl distamycin derivative of formula (I) is greatly enhanced when it is administeted in combination with an antibody inhibiting a growth factor or its receptor. The effect of the combined administration is significantly increased (synergic effect) with respect to the effect obtained administering each drug as single agent.

The present invention provides, in a first aspect, a combination comprising an acryloyl distamycin derivative of formula (I): 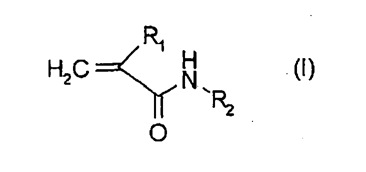 wherein:
R₁ is a bromine or chlorine atom;
R₂ is as defined in the description and in the claims; or a pharmaceutically acceptable salt thereof; and
   - an antibody inhibiting a growth factor or its receptor

The present invention includes, within its scope, the combination comprising any of the possible isomers covered by the compounds of formula (I), both considered separately or in admixture, as well as the metabolites and the pharmaceutically acceptable bioprecursors (otherwise known as pro-drugs) of the compounds of formula (I).

According to a preferred embodiment of the invention, the antibody inhibiting growth factor or its receptor is selected from Bevacizumab (antibody to vascular endothelial growth factor), Cetuximab, Panitumumab, Matuzumab, Nimotuzumab (antibodies to epidermal growth factor receptor), Trastuzumab and Pertuzumab (antibodies to ErbB2).

According to a more preferred embodiment of the invention, the antibody inhibiting growth factor or its receptor is Bevacizumab.

According to another preferred embodiment of the invention, herewith provided are the above combinations wherein, within the acryloyl distamycin derivative of formula (I), R₁ has the above reported meanings and R₂ is a group of formula (II) below: 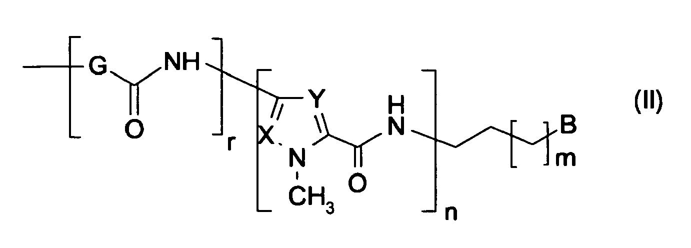 wherein
m is an integer from 0 to 2;
n is an integer from 2 to 5;
r is 0 or 1;
X and Y are, the same or different and independently for each heterocyclic ring, a nitrogen atom or a CH group;
G is phenylene, a 5 or 6 membered saturated or unsaturated heterocyclic ring with from 1 to 3 heteroatoms selected among N, O or S, or it is a group of formula (III) below: 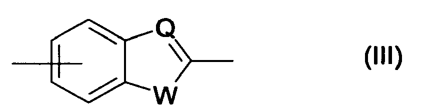
wherein Q is a nitrogen atom or a CH group and W is an oxygen or sulfur atom or it is a group NR₃ wherein R₃ is hydrogen or C₁-C₄ alkyl;
B is selected from the group consisting of

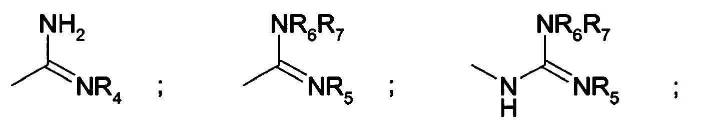

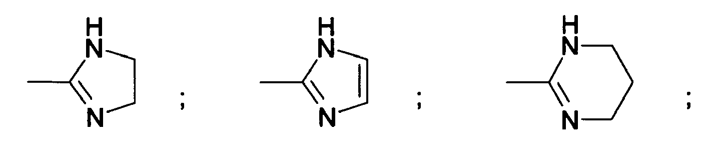

   C_{N} ; -NR₅R₆ - CONR₅R₆ ; - NHCONR₅R₆
wherein R₄ is cyano, amino, hydroxy or C₁-C₄ alkoxy; R₅, R₆ and R₇, the same or different, are hydrogen or C₁-C₄ alkyl.

In the present description, unless otherwise specified, with the term C₁-C₄ alkyl or alkoxy group we intend a straight or branched group selected from methyl, ethyl, n-propyl, isopropyl, n-butyl, isobutyl, sec-butyl, tert-butyl, methoxy, ethoxy, n-propoxy, isopropoxy, n-butoxy, isobutoxy, sec-butoxy or tert-butoxy.

Preferably, the combination of the invention comprise the above acryloyl distamycin derivative of formula (I) wherein R₁ is bromine or chlorine; R₂ is the above group of formula (II) wherein r is 0, m is 0 or 1, n is 4 and B has the above reported meanings. Still more preferred, within this class, are the combinations comprising the compounds of formula (I) wherein R₁ is bromine or chlorine; R₂ is the above group of formula (II) wherein r is 0, m is 0 or 1, n is 4, X and Y are both CH groups and B is selected from:

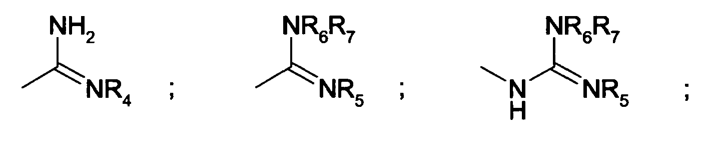

- CN ; - CONR₅R_{6 ;} - NHCONR₅R₆

wherein R₄ is cyano or hydroxy and R₅, R₆ and R₇, the same or different, are hydrogen or C₁-C₄ alkyl.

Even more preferred combination of the invention are those comprising a compound of formula (I) wherein R₁ is bromine, R₂ is the above group of formula (II) wherein r and m are 0, n is 4, X and Y are CH, B is a group of formula 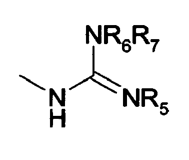 wherein R₅, R₆ and R₇ are hydrogen atoms, optionally in the form of a pharmaceutically acceptable salt thereof.

Pharmaceutically acceptable salts of the compounds of formula (I) are those with pharmaceutically acceptable inorganic or organic acids such as, for instance, hydrochloric, hydrobromic, sulfuric, nitric, acetic, propionic, succinic, malonic, citric, tartaric, methanesulfonic, p-toluenesulfonic acid and the like.

Examples of preferred acryloyl distamycin derivatives of formula (I), within the combination object of the invention, for instance in the form of pharmaceutically acceptable salts, preferably with hydrochloric acid, are:
1.N-(5-{[(5-{[(5-{[(2-{[amino(imino)methyl]amino}ethyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-4-[(2-bromoacryloyl)amino]-1-methyl-1H-pyrrole-2-carboxamide hydrochloride (Brostallicin);
2. N-(5-{[(5-{[(5-{[(2-{[amino(imino)methyl]amino}propyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-4-[(2-bromoacryloyl)amino]-1-methyl-1H-pyrrole-2-carboxamide hydrochloride;
3. N-(5-{[(5-{[(5-{[(3-ammo-3-iminopropyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-4-[(2-bromoacryloyl)amino]-1-methyl-1 H-pyrrole-2-carboxamide hydrochloride;
4. N-(5-{[(5-{[(5-{[(3-amino-3-iminopropyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-4-[(2-bromoacryloyl)amino]-1-methyl-1H-imidazole-2-carboxamide hydrochloride;
5. N-(5-{[(5-{[(5-{[(3-amino-3-iminopropyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-3-[(2-bromoacryloyl)amino]-1-methyl-1 H-pyrazole-5-carboxamide hydrochloride;
6. N-(5-{[(5-{[(5-{[(3-amino-3-oxopropyl)amino]carbonyl}-1-methyl-1H-pyrrol-3-yl)amino]carbonyl}-1-methyl-1H-pyrrol-3-yl)ammo]carbonyl}-1-methyl-1H-pyrrol-3-yl)-3-[(2-bromoacryloyl)amino]-1-methyl-1H-pyrazole-5-carboxamide;
7. N-(5-{[(5-{[(5-{[(2-{[amino(imino)methyl]amino}ethyl)amino]carbonyl}-1-methyl-1H-pyrrol-3-yl)amino]carbonyl}-1-methyl-1H-pyrrol-3-yl)amino]carbonyl} -1 - methyl-1H-pyrrol-3-yl)-4-[(2-chloroacryloyl)amino]-1-methyl-1H-pyrrole-2-carboxamide hydrochloride;
8. N-(5-{[(5-{[(3-{[amino(imino)methyl]amino}propyl)amino]carbonyl}-1-methyl-1H-pyrrol-3-yl)amino]carbonyl}-1-methyl-1H-pyrrol-3-yl)-4-[(2-bromoacryloyl)amino]-1-methyl-1H-pyrrole-2-carboxamidehydrochloride;
9. N-(5-{[(5-{[(3-amino-3-iminopropyl)amino]carbonyl}-1-methyl-1H-pyrrol-3-yl)amino]carbonyl}-1-methyl-1H-pyrrol-3-yl)-4-[(2-bromoacryloyl)amino]-1-methyl-1H-pyrrole-2-carboxamide hydrochloride; and
10. N-{5-[({5-[({5-[({3-[(aminocarbonyl)amino]propyl}amino)carbonyl]-1-methyl-1H-pyrrol-3-yl}amino)carbonyl]-1-methyl-1H-pyrrol-3-yl}amino)carbonyl]-1-methyl-1H-pyrrol-3-yl}-4-[(2-bromoacryloyl)amino]-1-methyl-1H-pyrrole-2-carboxamide.

Even more preferably the acryloyl distamycin derivatives of formula (I) is: N-(5-{[(5-{[(5-{[(2-{[amino(imino)methyl]amino}ethyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-4-[(2-bromoacryloyl)amino]-1-methyl-1H-pyrrole-2-carboxamide hydrochloride (Brostallicin) and the antibody inhibiting growth factor or its receptor is Bevacizumab.

The above compounds of formula (I), either specifically identified as such or by means of the general formula, are known or easily prepared according to known methods as reported, for instance, in the aforementioned international patent applications WO 90/11277, WO96/05196, WO 98/04524, WO 98/21202, WO 99/50265 and WO 99/50266 as well as in WO 01/40181.

Any of the combinations of an acryloyl distamycin derivative of formula (I), as defined above, and an antibody inhibiting growth factor or its receptor as listed above, are intended as fixed combination and for simultaneous, separate, or sequential use.

Another aspect of the present invention as listed above is to provide a combination of an acryloyl distamycin derivative of formula (I), as defined above, and an antibody inhibiting growth factor or its receptor as listed above, for use in the treatment of cancer in a subject in need thereof.

A further aspect of the present invention relates to the use of a combination of an acryloyl distamycin derivative of formula (I), as defined above, and an antibody inhibiting growth factor or its receptor as listed above, for the preparation of a medicament for use in the treatment of cancer in a subject in need thereof.

As used herein, the term "cancer" refers to all forms of cancer including cancer of blood and lymphatic systems comprising Hodgking's disease, leukemias, lymphomas, multiple myeloma and Waldenstrom's disease; skin cancer comprising malignant melanoma; cancers of digestive tract comprising head and neck, esophageal, stomach, pancreas, liver, colon and rectal and anal cancer; cancer of genital and urinary systems comprising kidney, bladder, testis and prostate cancer; gynecological cancers comprising cervical, endometrial, ovarian, fallopian tube, vaginal and vulvar cancer; breast cancer, brain cancer, bone cancer, carcinoid tumors, nasopharyngeal cancer, thyroid cancer, retroperitoneal sarcomas and soft tissue.

For example, the combined preparations, according to the invention, are directed to the treatment of head and neck cancer, colon and rectal cancer, retroperitoneal sarcomas and soft tissue.

As used herein, the terms "treatment" or "treating" or "to treat" mean to alleviate symptoms, eliminate the causation either on a temporary or permanent basis, or to prevent or slow the appearance of symptoms. The term "treatment" includes alleviation, elimination of causation of or prevention of cancer. Besides being useful for human treatment, these combinations are also useful for treatment of mammals, including horses, dogs, cats, rats, mice, sheep, pigs, etc.

The term "subject" for purposes of treatment includes any human or animal subject who is in need of the prevention of, or who has a cancer. The subject is typically a mammal. "Mammal", as that term is used herein, refers to any animal classified as a mammal, including humans, domestic and farm animals, and zoo, sports or pet animals, such as dogs, horses, cats, cattle, etc., Preferably, the mammal is a human.

By the term "synergistic antineoplastic effect", as used herein, it is meant the inhibition of the growth tumor, preferably the complete regression of the tumor, by administering an effective amount of the combination comprising an acryloyl distamycin derivative of formula (I), and an antibody inhibiting a growth factor or its receptor.

A further aspect of the present invention relates to the use of a combination of an acryloyl distamycin derivative of formula (I), as defined above, and an antibody inhibiting growth factor or its receptor as listed above, for the preparation of a medicament for the prevention or treatment of metastasis or the treatment of tumors by inhibition of angiogenesis.

The constituents of the combined preparations according to the invention can be administered to a patient in any acceptable manner that is medically acceptable including orally, parenterally, or with local therapeutic approaches such as, e.g., implants. Oral administration includes administering the constituents of the combined preparation in a suitable oral form such as, e.g., tablets, capsules, lozenges, suspensions, solutions, emulsions, powders, syrups and the like. Parenteral administration includes administering the constituents of the combined preparation by subcutaneous, intravenous or intramuscular injections. Local therapeutic approaches include implants, for example intra- arterial implants.

Typically a substituted acryloyl distamycin derivative of formula (I) is administered intravenously, typically an antibody inhibiting a growth factor or its receptor is administered intravenously or orally. The actual preferred dosage, method, order and time of administration of the constituents of the combined preparations of the invention may vary according to, inter alia, the particular pharmaceutical formulation of a substituted acryloyl distamycin derivative of formula (I) being utilized and the particular pharmaceutical formulation of an antibody inhibiting a growth factor or its receptor being utilized, the particular cancer being treated, the age, condition, sex and extent of the disease treated and can be determined by one of skill in the art.

The dosage regimen must therefore be tailored to the particular of the patient's conditions, response and associate treatments, in a manner, which is conventional for any therapy, and may need to be adjusted in response to changes in conditions and/or in light of other clinical conditions.

As a non limiting example, suitable dosages of the acryloyl-distamycin derivatives of formula (I) may range from about 0.05 mg/m2 to about 100 mg/m2 of body surface area and, more preferably, from about 0.1 to about 50 mg/m2 of body surface area.

For the administration of an antibody inhibiting a growth factor or its receptor, according to the method of the invention, the course of therapy generally employed may be from 0.1 mg/kg to 100 mg/kg . More preferably, the course of therapy employed is from about 1 mg/kg to 20 mg/kg.

When the active constituents of the combined preparation according to the invention are supplied along with a pharmaceutically acceptable carrier or excipient, a pharmaceutical composition is formed. Such pharmaceutical composition constitutes a further embodiment of the invention.

Pharmaceutically acceptable carriers and excipients are chosen such that side effects from the pharmaceutical compound are minimized and the performance of the compound is not cancelled or inhibited to such an extent that treatment is ineffective.

Pharmaceutically acceptable carriers or excipients to be utilized in the preparation of a pharmaceutical composition according to the invention are well known to people skilled in the art of formulating compounds in a form of pharmaceutical compositions. For example, "pharmaceutically acceptable carrier" refers to one or more compatible solid or liquid filler, diluent or encapsulating substances which are suitable for administration to mammals including humans. For example, "pharmaceutically acceptable excipient" refers to any inert substance used as a diluent or vehicle for an active substance(s) that is intentionally added to the formulation of a dosage form. The term includes binders, fillers' disintegrants, and lubricants.

Techniques for formulation and administration of drugs can be found in "Remington's Pharmacological Sciences"; Mack Publishing Co., Easton, PA., latest edition.

Pharmaceutical compositions suitable for parenteral administration are formulated in a sterile form The sterile composition thus may be a sterile solution or suspension in a non-toxic parenterally acceptable diluent or solvent.

The amount of an active ingredient contained in the pharmaceutical composition according to the invention may vary quite widely depending upon many factors such as, for example, the administration route and the vehicle.

As an example, the pharmaceutical compositions of the invention may contain from about 0.05 mg/m2 to about 100 mg/m2 of body surface area of a substituted acryloyl distamycin derivative of formula (I); and from 0.1 mg/kg to 100 mg/kg of an antibody inhibiting a growth factor or its receptor.

Pharmaceutical compositions according to the invention are useful in anticancer therapy.

The present invention further provides a commercial kit comprising, in a suitable container means, an acryloyl distamycin derivative of formula (I), as defined above, and an antibody inhibiting growth factor or its receptor. In a kit according to the invention an acryloyl distamycin derivative of formula (I), as defined above, and an antibody inhibiting growth factor or its receptor are present within a single container means or within distinct container means.

Another embodiment of the present invention is a commercial kit comprising a pharmaceutical composition as described above.

Kits according to the invention are intended for simultaneous, separate or sequential use in antitumor therapy.

Kits according to the invention are intended for use in anticancer therapy.

The synergistic antineoplastic effect of the combined preparations of the present invention is shown, for instance, by the following *in vivo* test which is intended to illustrate the present invention without posing any limitation to it.

### IN VIVO antitumor efficacy

Balb Nu/Nu, male mice, (athymic mice) from Harlan, Italy, were maintained in cages with paper filter covers, food and bedding sterilized and water acidified 2.5 x 10⁶ DU145 human prostate carcinoma cells (from American Type Culture Collection) were injected subcutaneously in athymic mice. This tumor model was selected because it was previously demonstrated that bevacizumab inhibits angiogenesis and growth of this model *in vivo* [see for reference, The Prostate 36:1-10, 1998]. The treatments started 6 days later when the tumors were palpable. All drugs were prepared immediately before use. Brostallicin treatments were administered intravenously in a volume of 10 ml/kg at a dose of 0.2 mg/kg and treatments were repeated weekly for 3 weeks. Bevacizumab was administered intraperitoneally in a volume of 10 ml/kg at a dose of 20 mg/kg on the days 6,10,13,17,20 and 24 from the day of cell injection. When both compounds were administered on the same day, bevacizumab was administered intraperitoneally immediately before brostallicin intravenous injection. Every 3 days the tumor growth and the net body weight were evaluated. Tumor growth was assessed by caliper. The two diameters were recorded, and the tumor weight was calculated according to the following formula: length (mm) x width² (mm)/2. The effect of the antitumor treatment was determined as the delay in the onset of an exponential growth of tumors [see for reference, Anti Cancer Drugs 7: 437-60, 1996]. This delay (T-C value) was defined as the difference of the time (in days) required for the treatment group (T) and the control group (C) tumors to reach a predetermined size (e.g. 1 g). Toxicity was evaluated on the basis of the body weight reduction.

The results were shown in Table 2. Brostallicin combined with bevacizumab produced a strong synergistic effect: the T-Cs was significantly higher than that expected by the simple addition of the T-Cs obtained with the two compounds as single agent (21.2 days when the expected T-Cs was 14.2 days). No toxicity, also in terms of net body weight decrease, was observed within any treatment group.

**Table 2: In vivo antitumor efficacy**

| treatment | Time to reach 1 g (days) | T-C(days) | Toxicity |
|---|---|---|---|
| Control | 13.8 ±1.7 | | 0/8 |
| Bevacizumab 20 mg/kg * | 23.4 ± 4.6 | 9.6 | 0/8 |
| Brostallicin 0.2 mg/kg** | 18.4 ± 5.6 | 4.6 | 0/8 |
| Bevacizumab 20 mg/kg + Brostallicin 0.2mg/kg*** | 35.0 ± 8.6 | 21.2 | 0/8 |

| | | | |
|---|---|---|---|
| * Treatments administered intraperitoneally on day 6,10,13,17,20 and 24 ** Treatments administered intravenously on day 6, 13 and 20 (q7d x 3) *** Day 6, 13, 20: bevacizumab was injected intraperitoneally and immediately after brostallicin was injected intravenously; Day 10, 17, 24: mice were treated only with bevacizumab. | | | |

## Claims

1. A combination comprising an acryloyl distamycin derivative of formula (I) 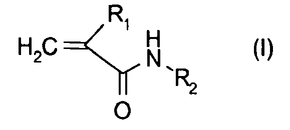 wherein:
R₁ is a bromine or chlorine atom;
R₂ is a group of formula (II) 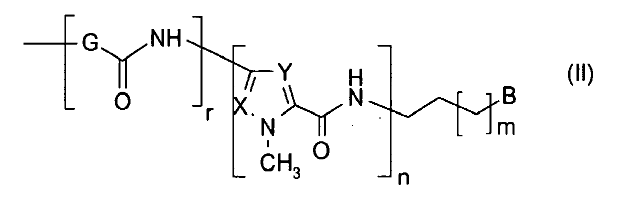
wherein
m is an integer from 0 to 2;
n is an integer from 2 to 5;
r is 0 or 1;
X and Y are, the same or different and independently for each heterocyclic ring, a nitrogen atom or a CH group;
G is phenylene, a 5 or 6 membered saturated or unsaturated heterocyclic ring with from 1 to 3 heteroatoms selected among N, O or S, or it is a group of formula (III) below: 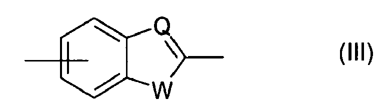
wherein Q is a nitrogen atom or a CH group and W is an oxygen or sulfur atom or it is a group NR₃ wherein R₃ is hydrogen or C₁-C₄ alkyl;
B is selected from the group consisting of 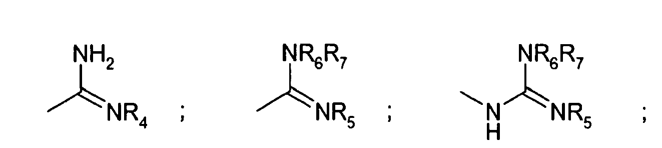 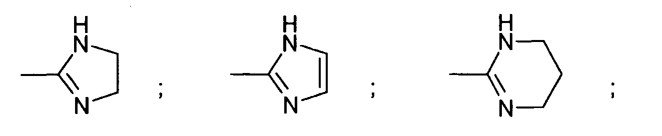
- CN -NR₅R₆ ; -CONR₅R₆; -NHCONR₅R₆
wherein R₄ is cyano, amino, hydroxy or C₁-C₄ alkoxy; R₅, R₆ and R₇, the same or different, are hydrogen or C₁-C₄ alky; and
- an antibody inhibiting a growth factor or its receptor

2. A combination according to claim 1 wherein the antibody inhibiting growth factor or its receptor is selected from the group consisting of Bevacizumab, Cetuximab, Panitumumab, Matuzumab, Nimotuzumab, Trastuzumab, and Pertuzumab.

3. A combination according to claim 2 wherein the antibody inhibiting growth factor or its receptor is Bevacizumab.

4. A combination according to claim 1 comprising an acryloyl distamycin derivative of formula (1) wherein R₂ is a group of formula (II) wherein:
m is 0 or 1, n is 4, r is 0, X and Y are both CH groups and B is selected from 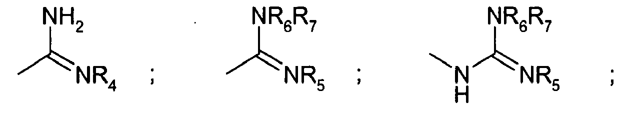
-CN ; -CONR₅R_{6 ;} -NHCONR₅R₆
wherein R₄ is cyano or hydroxy and R₅, R₆ and R₇, the same or different, are hydrogen or C₁-C₄ alkyl.

5. A combination according to claim 4 comprising an acryloyl distamycin derivative of formula (I) wherein:
R₁ is bromine, R₂ is a group of formula (II) wherein:
m is 0 and B is a group of formula 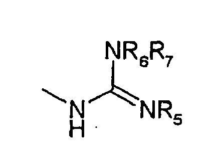
wherein R₅, R₆ and R₇ are hydrogen atoms, optionally in the form of a pharmaceutically acceptable salt;

6. A combination comprising
- N-(5-{[(S-{[(5-{[(2-{[amino(imino)methyl]amino}ethyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-4-[(2-bromoacryloyl)amino]-1-methyl-1H-pyrrole-2-carboxamide hydrochloride (Brostallicin); and
- Bevacizumab.

7. A combination according to anyone of claims 1 to 6 for use in the treatment of cancer in a subject in need thereof.

8. Use of a combination according to anyone of claims 1 to 7 in the preparation of a medicament for use in the treatment of cancer in a subject in need thereof.

9. Use of a combination according to anyone of claims 1 to 7 in the preparation of a medicament for the prevention or treatment of metastasis or the treatment of tumors by inhibition of angiogenesis.

10. A pharmaceutical composition comprising a combination according to anyone of the claims 1 to 7 and at least one pharmaceutically acceptable carrier or excipient.

11. A commercial kit comprising a combination according to anyone of the claims 1 to 7 or a pharmaceutical composition according to claim 10 for simultaneous, separate or sequential use in antitumor therapy.

## Patentansprüche

1. Kombination, welche ein Acryloyldistamycin-Derivat der Formel (I) 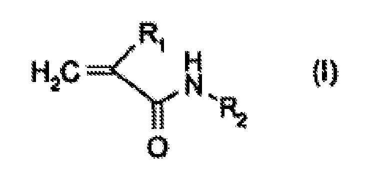 umfasst, wobei:
R₁ für ein Brom- oder Chlor-Atom steht;
R₂ für eine Gruppe der Formel (II) 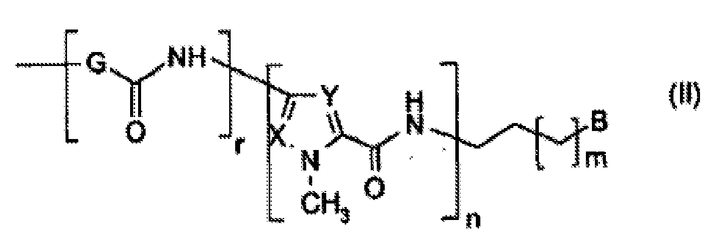
steht, wobei
m für eine ganze Zahl von 0 bis 2 steht;
n für eine ganze Zahl von 2 bis 5 steht;
r für 0 oder 1 steht;
X und Y für dieselben oder unterschiedliche Gruppen stehen und für jeden heterocyclischen Ring unabhängig voneinander für ein Stickstoffatom oder eine CH-Gruppe stehen;
G für eine Phenylengruppe, für einen 5- oder 6-gliedrigen gesättigten oder ungesättigten heterocyclischen Ring mit 1 bis 3 Heteroatomen, die aus N, O und S ausgewählt sind, oder für eine Gruppe der Formel (III) 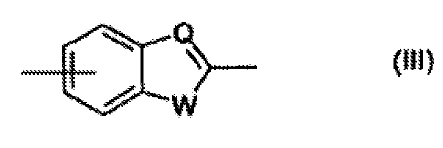
steht, wobei Q für ein Stickstoffatom oder eine CH-Gruppe steht und W für ein Sauerstoff- oder Schwefelatom oder für eine NR₃-Gruppe steht, wobei R₃ für Wasserstoff oder eine C₁-C₄-Alkylgruppe steht;
B aus der Gruppe ausgewählt ist, die aus 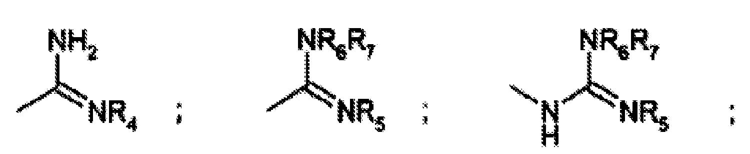 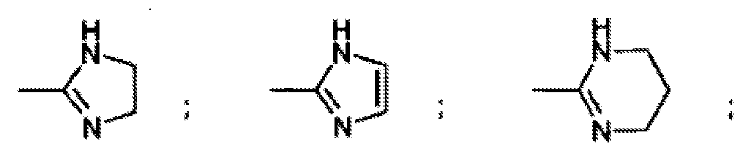
-CN ; -NR₅R₆ ; CONR₅R₆ ; -NHCONR₅R₆
besteht, wobei R₄ für eine Cyano-, Amino-, Hydroxy- oder C₁-C₄-Alkoxygruppe steht; R₅, R₆ und R₇ für dieselben oder unterschiedliche Gruppen stehen und für Wasserstoff oder eine C₁-C₄-Alkylgruppe stehen; und
einen Antikörper umfasst, der einen Wachstumsfaktor oder dessen Rezeptor inhibiert.

2. Kombination nach Anspruch 1, wobei der Antikörper, der einen Wachstumsfaktor oder dessen Rezeptor inhibiert, aus der Gruppe ausgewählt ist, die aus Bevacizumab, Cetuximab, Panitumumab, Matuzumab, Nimotuzumab, Trastuzumab und Pertuzumab besteht.

3. Kombination nach Anspruch 2, wobei es sich bei dem Antikörper, der einen Wachstumsfaktor oder dessen Rezeptor inhibiert, um Bevacizumab handelt.

4. Kombination nach Anspruch 1, welche ein Acryloyldistamycin-Derivat der Formel (I) umfasst, wobei R₂ für eine Gruppe der Formel (II) steht, wobei m für 0 oder 1 steht, n für 4 steht, r für 0 steht, X und Y beide für CH-Gruppen stehen und B aus 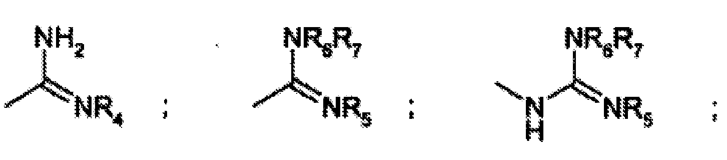
-CN ; -CONR₅R₆ ; -NHCONR₅R₆
ausgewählt ist, wobei R₄ für eine Cyano- oder Hydroxygruppe steht und R₅, R₆ und R₇ für dieselben oder unterschiedliche Gruppen stehen und für Wasserstoff oder eine C₁-C₄-Alkylgruppe stehen.

5. Kombination nach Anspruch 4, welche ein Acryloyldistamycin-Derivat der Formel (I) umfasst, wobei
R₁ für Brom steht, R₂ für eine Gruppe der Formel (II) steht, wobei
m für 0 steht und B für eine Gruppe der Formel 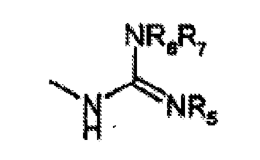 steht, wobei R₅, R₆ und R₇ für Wasserstoffatome stehen, gegebenenfalls in der Form eines pharmazeutisch unbedenklichen Salzes.

6. Kombination, welche das Folgende umfasst:
- N-(5-{[(5-{[(5-{[(2-{[Amino(imino)methyl]amino}ethyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-4-[(2-bromacryloyl)amino]-1-methyl-1H-pyrrol-2-carboxamid-Hydrochlorid (Brostallicin) und
- Bevacizumab.

7. Kombination nach einem der Ansprüche 1 bis 6 zur Verwendung bei der Behandlung von Krebs bei einem Behandlungssubjekt, das sie benötigt.

8. Verwendung einer Kombination nach einem der Ansprüche 1 bis 7 bei der Herstellung eines Medikaments zur Verwendung bei der Behandlung von Krebs bei einem Behandlungssubjekt, das sie benötigt.

9. Verwendung einer Kombination nach einem der Ansprüche 1 bis 7 bei der Herstellung eines Medikaments zur Prävention oder Behandlung von Metastasen oder zur Behandlung von Tumoren durch Inhibition der Angiogenese.

10. Pharmazeutische Zusammensetzung, welche eine Kombination gemäß einem der Ansprüche 1 bis 7 und mindestens einen pharmazeutisch unbedenklichen Träger- oder Hilfsstoff umfasst.

11. Kommerzieller Kit, welcher eine Kombination nach einem der Ansprüche 1 bis 7 oder eine pharmazeutische Zusammensetzung nach Anspruch 10 zur gleichzeitigen, getrennten oder sequenziellen Anwendung in der Antitumortherapie umfasst.

## Revendications

1. Combinaison comprenant un dérivé de distamycine d'acryloyle de formule (I) 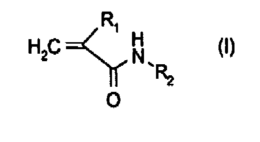 où
R₁ est un atome de brome ou de chlore ;
R₂ est un groupe de formule (II) 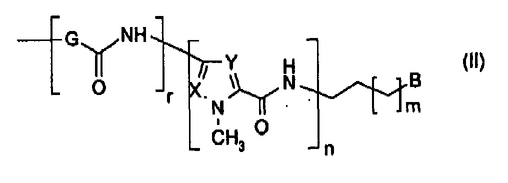
où
m est un nombre entier compris entre 0 et 2 ;
n est un nombre entier compris entre 2 et 5 ;
r vaut 0 ou 1 ;
X et Y, identiques ou différents, sont indépendamment l'un de l'autre, pour chaque anneau hétérocyclique, un atome d'azote ou un groupe CH ;
G est du phénylène, un anneau hétérocyclique saturé ou insaturé à 5 ou 6 chaînons ayant entre 1 et 3 hétéroatomes choisis parmi N, O ou S, ou est un groupe de formule (III) ci-dessous : 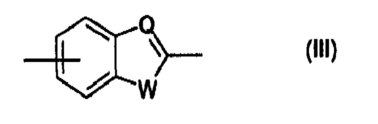
dans laquelle Q est un atome d'azote ou un groupe CH et W est un atome d'oxygène ou de soufre ou est un groupe NR₃ où R₃ est de l'hydrogène ou un alkyle en C₁-C₄ ;
B est sélectionné dans le groupe constitué par 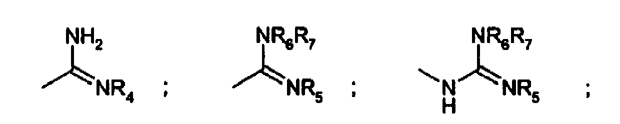 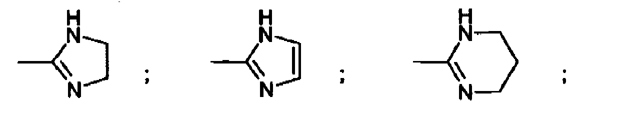
—CN ; —NR₅R₆ ; —CONR₅R₆ ; —NHCONR₅R₆
où R₄ est un cyano, amino, hydroxy ou alcoxy en C₁-C₄ ; R₅, R₆ et R₇, identiques ou différents, sont de l'hydrogène ou un alkyle en C₁-C₄ ; et
- un anticorps inhibant un facteur de croissance ou son récepteur.

2. Combinaison selon la revendication 1, dans laquelle l'anticorps inhibant un facteur de croissance ou son récepteur est sélectionné dans le groupe constitué par le Bevacizumab, le Cetuximab, le Panitumumab, le Matuzumab, le Nimotuzumab, le Trastuzumab et le Pertuzumab.

3. Combinaison selon la revendication 2, dans laquelle l'anticorps inhibant un facteur de croissance ou son récepteur est le Bevacizumab.

4. Combinaison selon la revendication 1, comprenant un dérivé de distamycine d'acryloyle de formule (I) où R₂ est un groupe de formule (II) où :
m vaut 0 ou 1, n vaut 4, r vaut 0, X et Y sont tous deux des groupes CH et B est sélectionné parmi 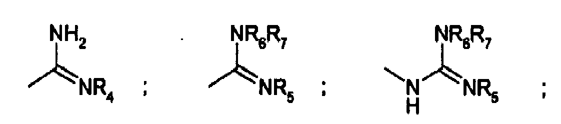
—CN ; —CONR₅R₆ ; —NHCONR₅R₆
où R₄ est un cyano ou un hydroxy et R₅, R₆ et R₇, identiques ou différents, sont de l'hydrogène ou un alkyle en C₁-C₄.

5. Combinaison selon la revendication 4, comprenant un dérivé de distamycine d'acryloyle de formule (I) où :
R₁ est du bromure, R₂ est un groupe de formule (II) où :
m vaut 0 et B est un groupe de formule 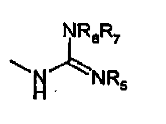
où R₅, R₆ et R₇ sont des atomes d'hydrogène, facultativement sous la forme d'un sel pharmaceutiquement acceptable.

6. Combinaison comprenant
- du chlorhydrate de N-(5-{[(5-{[(5-{[(2-{[amino(imino)méthyl]amino}éthyl)amino]carbonyl}-1-méthyl-1H-pyrrol-3-yl)amino]carbonyl}-1-méthyl-1H-pyrrol-3-yl)amino]carbonyl}-1-méthyl-1H-pyrrol-3-yl)-4-[(2-bromoacryloyl)amino]-1-méthyl-1H-pyrrole-2-carboxamide (Brostallicine) ; et
- du Bevacizumab.

7. Combinaison selon l'une quelconque des revendications 1 à 6, pour une utilisation dans le traitement du cancer chez un patient en ayant besoin.

8. Utilisation d'une combinaison selon l'une quelconque des revendications 1 à 7, dans la préparation d'un médicament destiné à être utilisé dans le traitement du cancer chez un patient en ayant besoin.

9. Utilisation d'une combinaison selon l'une quelconque des revendications 1 à 7, dans la préparation d'un médicament destiné à être utilisé pour la prévention ou le traitement des métastases ou le traitement des tumeurs par inhibition de l'angiogenèse.

10. Composition pharmaceutique comprenant une combinaison selon l'une quelconque des revendications 1 à 7, et au moins un support ou excipient pharmaceutiquement acceptable.

11. Kit commercial comprenant une combinaison selon l'une quelconque des revendications 1 à 7 ou une composition pharmaceutique selon la revendication 10, pour l'utilisation simultanée, séparée ou séquentielle dans le cadre d'une thérapie antitumorale.
